# EUROPEAN PATENT APPLICATION

(11) **EP 4 049 691 A1**
(43) Date of publication of application: **31.08.2022**
(21) Application number: 20879436.2
(22) Date of filing: 11.06.2020
(51) Int. Cl.: A61L 101/06, B65D 77/04, C01B 11/02, A01N 59/00, A01P 1/00, A61L 2/20, B65D 81/28, A01N 25/34, A01P 3/00, A61L 9/01, A61L 9/12

(54) **BACTERICIDE DIFFUSION DEVICE AND DIFFUSION METHOD**

(30) Priority: 23.10.2019 JP 2019192738; 20.01.2020 JP 2020006565
(71) Applicant: Amatera, Inc., Aichi-gun, Aichi 470-0161 (JP)
(72) Inventor: FUJITA, Hiromasa, Aichi-gun, Aichi 470-0161 (JP)
(74) Representative: Vossius & Partner Patentanwälte Rechtsanwälte mbB
(86) International application number: PCT/JP2020/023060
(87) International publication number: WO 2021/079555

(57) **Abstract**

There are provided a bactericide diffusion apparatus and a bactericide diffusion method that have excellent design and make it possible to stably diffuse a bactericide. A bactericide diffusion apparatus (10) includes: a bactericide containing chlorine dioxide; a first container (130) housing the bactericide and having a first opening; and a second container (100) housing the first container (130), having a second opening and being made of a ceramic. The first container (130) includes a first portion defining the first opening, a second portion located below the first portion and having a diameter larger than that of the first opening, and a third portion located below the second portion and having a diameter larger than that of the second portion. The second container (100) includes a main body portion (120) housing the first container (130) and having the second opening, and a lid portion (110) fitted to the second opening. The lid portion (110) has a hole (111) for diffusing chlorine dioxide.

## Description

### TECHNICAL FIELD

The present invention relates to a container for diffusing a chemical substance such as chlorine dioxide, which is widely used for treatment of an allergy-inducing substance such as a pollen, dust, skin flake and fungi, treatment of a harmful substance such as a pathogenic bacteria, virus and a harmful chemical substance (for example, tobacco smoke, and formaldehyde), environmental purification, deodorization of indoor/outdoor and a food product, fungusproofing and antisepsis, etc. The present application claims the priority based on Japanese Patent Application No. 2019-192738 filed on October 23, 2019 and Japanese Patent Application No. 2020-006565 filed on January 20, 2020, the entire contents of which are incorporated herein by reference.

### BACKGROUND ART

Since chlorine dioxide (ClO₂) has strong oxidizing power, it is widely used for treatment of an allergy-inducing substance such as a pollen, dust, skin flake and fungi, treatment of a harmful substance such as a pathogenic bacteria, virus and a harmful chemical substance (for example, tobacco smoke, and formaldehyde), environmental purification, deodorization of indoor/outdoor and a food product, mildewproofing and antisepsis, etc. A composition containing useful chlorine dioxide which is used for such a wide range of applications and a method for producing the same have been proposed.

For example, Japanese Patent Laying-Open No. 11-278808 (PTL 1) proposes a pure chlorine dioxide solution having dissolved chlorine dioxide gas, a chlorite and a pH adjuster as constituents, a gel composition containing the above pure chlorine dioxide solution and a highly water-absorbing resin, a foamable composition containing the above pure chlorine dioxide solution and a foaming agent, and a container for containing the above pure chlorine dioxide solution, the above gel composition, and the above foamable composition.

WO 2008/111358 (PTL 2) proposes a stabilized composition for chlorine dioxide capable of maintaining the concentration of chlorine dioxide in an agent substantially constant even when chlorine dioxide is continuously released as gas from the agent containing dissolved chlorine dioxide, wherein the composition comprises a combination of a chlorite and a pH adjuster, and the pH adjuster is an acid having buffering properties by which the pH of 5% aqueous solution at 25°C is adjusted to 2.5 to 6.8, or a salt thereof.

### CITATION LIST

### PATENT LITERATURE

PTL 1: Japanese Patent Laying-Open No. 11-278808
PTL 2: WO 2008/111358

### SUMMARY OF INVENTION

### TECHNICAL PROBLEM

The conventional technique had a problem of poor design because a container that housed a bactericide containing chlorine dioxide was exposed. Furthermore, the conventional technique had a problem that stable diffusion of a bactericide was difficult.

Thus, the present invention has been made to solve the above-described problem, and an object of the present invention is to provide a bactericide diffusion apparatus and a bactericide diffusion method that have excellent design and make it possible to stably diffuse a bactericide.

### SOLUTION TO PROBLEM

A bactericide diffusion apparatus according to one aspect includes: a bactericide containing chlorine dioxide; a first container housing the bactericide and having a first opening; and a second container housing the first container, having a second opening and being made of a ceramic, wherein the first container includes a first portion defining the first opening, a second portion located below the first portion and having a diameter larger than that of the first opening, and a third portion located below the second portion and having a diameter larger than that of the second portion, the second container includes a main body portion housing the first container and having the second opening, and a lid portion fitted to the second opening, and the lid portion has a hole for diffusing chlorine dioxide.

According to the bactericide diffusion apparatus configured as described above, the first container housing the bactericide includes the first portion defining the opening portion, the second portion located below the first container and having a diameter larger than that of the opening, and the third portion located below the second portion and having a large diameter. Therefore, the bactericide stored in the third portion is narrowed by the second portion and the first opening each having a small diameter, and thus, diffuses gradually. As a result, the bactericide can be stably diffused. Furthermore, since the first container is housed in the second container made of a ceramic, it is difficult to see the first container from the outside, and thus, the design of the bactericide diffusion apparatus can be improved.

A bactericide diffusion apparatus according to another aspect includes: a bactericide containing chlorine dioxide; a first container housing the bactericide and having a first opening; and a second container housing the first container, having a second opening and being made of a ceramic, wherein the first container includes a first portion defining the first opening, a second portion located below the first portion and having a diameter larger than that of the first opening, and a third portion located below the second portion and having a diameter larger than that of the second portion, the second container includes a main body portion housing the first container and having the second opening, and chlorine dioxide diffuses from the second opening to the outside.

According to the bactericide diffusion apparatus configured as described above, the first container housing the bactericide includes the first portion defining the opening portion, the second portion located below the first container and having a diameter larger than that of the opening, and the third portion located below the second portion and having a large diameter. Therefore, the bactericide stored in the third portion is narrowed by the second portion and the first opening each having a small diameter, and thus, diffuses gradually. As a result, the bactericide can be stably diffused. Furthermore, since the first container is housed in the second container made of a ceramic, it is difficult to see the first container from the outside, and thus, the design of the bactericide diffusion apparatus can be improved.

Preferably, the first container includes: a first reduced-diameter portion located above the second portion and having an inner diameter that becomes smaller toward the first opening; a first increased-diameter portion located below the second portion and having an inner diameter that becomes larger toward the first opening; a first cylindrical portion located between the first reduced-diameter portion and the first increased-diameter portion and having a cylindrical shape; a second reduced-diameter portion located above the third portion and having an inner diameter that becomes smaller toward the first opening; a second increased-diameter portion located below the third portion and having an inner diameter that becomes larger toward the first opening; and a second cylindrical portion located between the second reduced-diameter portion and the second increased-diameter portion and having a cylindrical shape.

In the bactericide diffusion apparatus configured as described above, each of the first portion and the second portion includes the reduced-diameter portion and the increased-diameter portion, and thus, the first portion and the second portion have a barrel shape and the strength is improved as compared with the case in which all of the first portion and the second portion have a cylindrical shape. Furthermore, a person can easily hold a portion between the first reduced-diameter portion of the first portion and the second increased-diameter portion of the second portion.

A bactericide diffusion method using any bactericide diffusion apparatus described above includes: adjusting an amount of diffusion of the bactericide from the first opening by adjusting a degree of opening of the first opening; and housing the first container in the second container.

In the bactericide diffusion method configured as described above, the bactericide can be reliably diffused, and further, the bactericide diffusion method that does not impair the design can be provided.

### ADVANTAGEOUS EFFECTS OF INVENTION

There can be provided a bactericide diffusion apparatus and a bactericide diffusion method that have excellent design and make it possible to stably diffuse a bactericide.

### BRIEF DESCRIPTION OF DRAWINGS

Fig. 1 is a perspective view of a bactericide diffusion apparatus 10 according to a first embodiment.
Fig. 2 is a cross-sectional view of a second container 100 taken along line II-II in Fig. 1.
Fig. 3 shows an internal structure of bactericide diffusion apparatus 10 according to the first embodiment shown in Figs. 1 and 2.
Fig. 4 is a perspective view of a first container 130 used in the first embodiment.
Fig. 5 is a cross-sectional view of first container 130 taken along line V-V in Fig. 4.
Fig. 6 is a perspective view of first container 130 used in a second embodiment.
Fig. 7 is a cross-sectional view of first container 130 taken along line VII-VII in Fig. 6.
Fig. 8 is a perspective view of first container 130 used in the first embodiment.
Fig. 9 is a cross-sectional view of first container 130 taken along line IX-IX in Fig. 8.

### DESCRIPTION OF EMBODIMENTS

### (First Embodiment)

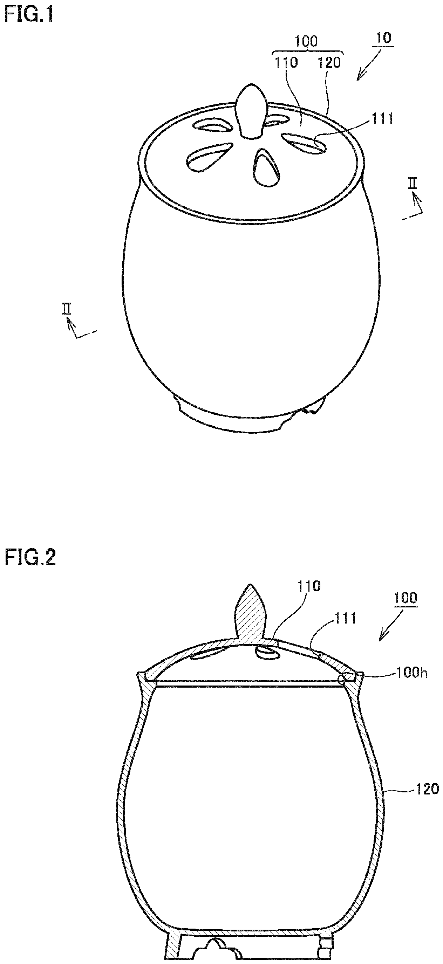

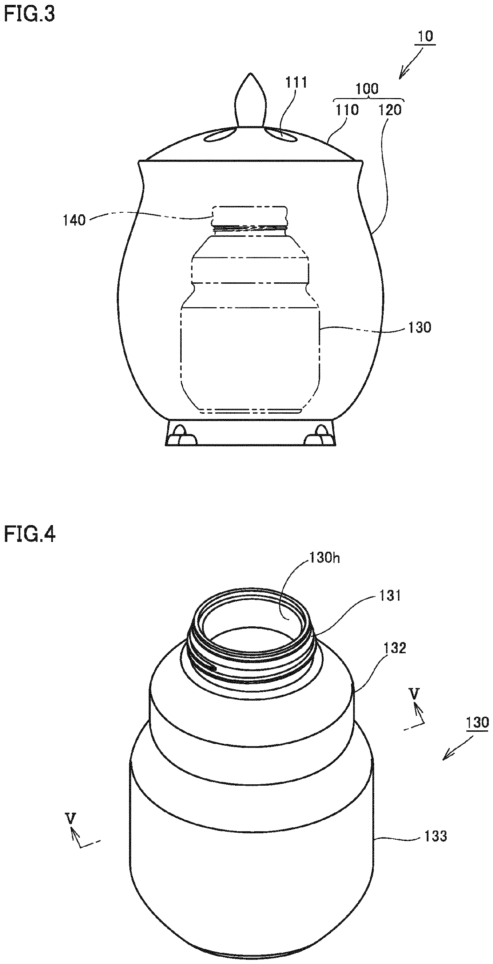

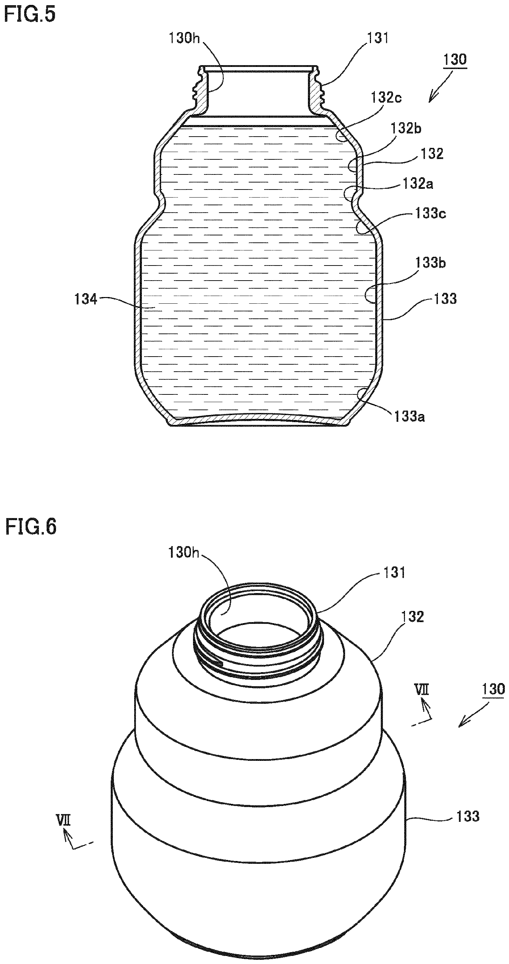

Fig. 1 is a perspective view of a bactericide diffusion apparatus 10 according to a first embodiment. Fig. 2 is a cross-sectional view of a second container 100 taken along line II-II in Fig. 1. Fig. 3 shows an internal structure of bactericide diffusion apparatus 10 according to the first embodiment shown in Figs. 1 and 2. Fig. 4 is a perspective view of a first container 130 used in the first embodiment. Fig. 5 is a cross-sectional view of first container 130 taken along line V-V in Fig. 4.

As shown in Figs. 1 to 5, bactericide diffusion apparatus 10 according to the first embodiment includes a bactericide 134 containing chlorine dioxide, a first container 130 housing bactericide 134 and having a first opening 130h, and a second container 100 housing first container 130, having a second opening 100h and being made of a ceramic.

First container 130 includes a first portion 131 defining first opening 130h, a second portion 132 located below first portion 131 and having a diameter larger than that of first opening 130h, and a third portion 133 located below second portion 132 and having a diameter larger than that of second portion 132. Second container 100 includes a main body portion 120 housing first container 130 and having second opening 100h, and a lid portion 110 fitted to second opening 100h. Lid portion 110 has a hole 111 for diffusing chlorine dioxide.

First container 130 includes a first reduced-diameter portion 132c located above second portion 132 and having an inner diameter that becomes smaller toward first opening 130h, a first increased-diameter portion 132a located below second portion 132 and having an inner diameter that becomes larger toward first opening 130h, a first cylindrical portion 132b located between first reduced-diameter portion 132c and first increased-diameter portion 132a and having a cylindrical shape, a second reduced-diameter portion 133c located above third portion 133 and having an inner diameter that becomes smaller toward first opening 130h, a second increased-diameter portion 133a located below third portion 133 and having an inner diameter that becomes larger toward first opening 130h, and a second cylindrical portion 133b located between second reduced-diameter portion 133c and second increased-diameter portion 133a and having a cylindrical shape.

First container 130 is made of plastic. First container 130 is made of, for example, plastic that does not react with chlorine dioxide contained in bactericide 134. A thread ridge is formed on an outer circumference of first portion 131 of first container 130. The thread ridge and a thread groove in an inner circumferential surface of a lid 140 are fitted to each other.

By changing an opening area of lid 140, an amount of diffusion of chlorine dioxide from first container 130 can be adjusted. By making the opening area of lid 140 variable, the opening area can be changed. In order to diffuse a maximum amount of bactericide 134 from first container 130, bactericide diffusion apparatus 10 can be used with lid 140 removed. Lid 140 does not need to be provided from the beginning.

Chlorine dioxide as bactericide 134 may be in various forms such as solid, liquid, gel, and sol. Since chlorine dioxide kills a virus because of the oxidizing power thereof, chlorine dioxide functions as bactericide 134. Bactericide 134 may contain a substance other than chlorine dioxide, e.g., a substance for making chlorine dioxide exist stably.

Since first container 130 includes second increased-diameter portion 133a, an area of contact between bactericide 134 and the air can be increased even when only a small amount of bactericide 134 is present in first container 130. Therefore, diffusion of bactericide 134 can be promoted.

The presence of second reduced-diameter portion 133c and first increased-diameter portion 132a makes it easier for a person to hold a recessed portion provided between second reduced-diameter portion 133c and first increased-diameter portion 132a. Furthermore, the presence of the recessed portion increases the strength of first container 130.

Second container 100 is made of a ceramic. Since second container 100 is made of a ceramic, a pattern can be formed on an outer surface of second container 100. As a result, the design of bactericide diffusion apparatus 10 can be improved. Since second container 100 is made of a ceramic, second container 100 can prevent oxidation of chlorine dioxide. Even if bactericide 134 spills in second container 100, bactericide 134 does not corrode second container 100. It is preferable that an inner circumferential surface of second container 100 be covered with a coating that is not corroded by chlorine dioxide. Furthermore, it is preferable that the inner circumferential surface of second container 100 have no hole in order to prevent chlorine dioxide from permeating second container 100.

An upper end of main body portion 120 of second container 100 has a flange shape that protrudes inward. An inner circumferential surface of the flange defines second opening 100h. By forming the flange, the strength of second opening 100h can be improved.

Lid portion 110 is placed on the flange. Lid portion 110 has five holes 111 radially extending in the direction of the radius. The number of holes 111 is not limited to five, and more or less holes 111 may be provided.

Main body portion 120 has such a shape that an inner diameter thereof becomes larger gradually from the bottom upward, and becomes smaller gradually from a certain portion. This makes it easier for a person to hold first container 130 and place first container 130 in main body portion 120.

### (Second Embodiment)

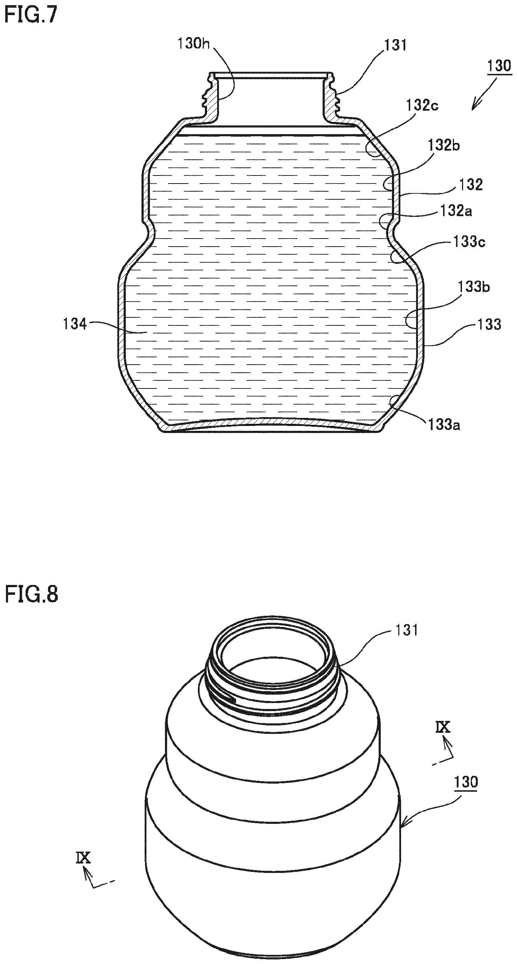

Fig. 6 is a perspective view of first container 130 used in a second embodiment. Fig. 7 is a cross-sectional view of first container 130 taken along line VII-VII in Fig. 6. As shown in Figs. 6 and 7, first container 130 according to the second embodiment has a greater breadth than that of first container 130 according to the first embodiment. Therefore, a larger amount of bactericide 134 is stored in first container 130.

### (Third Embodiment)

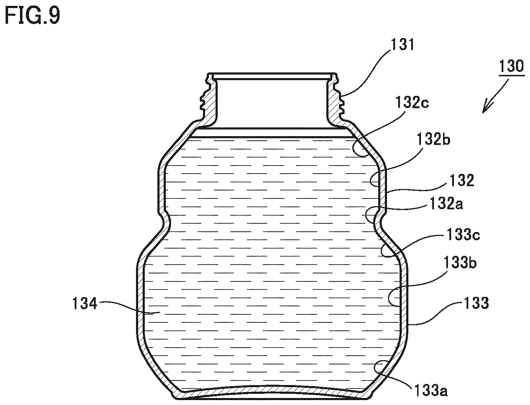

Fig. 8 is a perspective view of first container 130 used in a third embodiment. Fig. 9 is a cross-sectional view of first container 130 taken along line IX-IX in Fig. 8. As shown in Figs. 8 and 9, first container 130 according to the third embodiment is smaller than first container 130 according to the first embodiment. Therefore, it is preferable to house first container 130 according to the third embodiment in small second container 100.

In a diffusion method for bactericide 134, bactericide 134 is housed in first container 130. A diffusion speed of bactericide 134 from first container 130 is determined by an opening area of first opening 130h and a concentration of bactericide 134 outside first container 130 and inside second container 100. Since the concentration of bactericide 134 inside second container 100 is set substantially constant, the diffusion speed of bactericide 134 from first container 130 can be determined by adjusting the opening area of first opening 130h.

A bactericide diffusion method using diffusion apparatus 10 for bactericide 134 according to an embodiment includes adjusting an amount of diffusion of bactericide 134 from first opening 130h by adjusting a degree of opening of first opening 130h, and housing first container 130 in second container 100.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

### INDUSTRIAL APPLICABILITY

The present invention can be used in the field of a bactericide diffusion apparatus and a bactericide diffusion method.

### REFERENCE SIGNS LIST

10 diffusion apparatus; 100 second container; 100h second opening; 110 lid portion; 111 hole; 120 main body portion; 130 first container; 130h first opening; 131 first portion; 132 second portion; 132a first increased-diameter portion; 132b first cylindrical portion; 132c first reduced-diameter portion; 133 third portion; 133a second increased-diameter portion; 133b second cylindrical portion; 133c second reduced-diameter portion; 134 bactericide; 140 lid.

## Claims

1. A bactericide diffusion apparatus comprising:
a bactericide containing chlorine dioxide;
a first container housing the bactericide and having a first opening; and
a second container housing the first container, having a second opening and being made of a ceramic, wherein
the first container includes a first portion defining the first opening, a second portion located below the first portion and having a diameter larger than that of the first opening, and a third portion located below the second portion and having a diameter larger than that of the second portion,
the second container includes a main body portion housing the first container and having the second opening, and a lid portion fitted to the second opening, and
the lid portion has a hole for diffusing chlorine dioxide.

2. A bactericide diffusion apparatus comprising:
a bactericide containing chlorine dioxide;
a first container housing the bactericide and having a first opening; and
a second container housing the first container, having a second opening and being made of a ceramic, wherein
the first container includes a first portion defining the first opening, a second portion located below the first portion and having a diameter larger than that of the first opening, and a third portion located below the second portion and having a diameter larger than that of the second portion,
the second container includes a main body portion housing the first container and having the second opening, and
chlorine dioxide diffuses from the second opening to the outside.

3. The bactericide diffusion apparatus according to claim 1 or 2, wherein
the first container includes:
a first reduced-diameter portion located above the second portion and having an inner diameter that becomes smaller toward the first opening;
a first increased-diameter portion located below the second portion and having an inner diameter that becomes larger toward the first opening;
a first cylindrical portion located between the first reduced-diameter portion and the first increased-diameter portion and having a cylindrical shape;
a second reduced-diameter portion located above the third portion and having an inner diameter that becomes smaller toward the first opening;
a second increased-diameter portion located below the third portion and having an inner diameter that becomes larger toward the first opening; and
a second cylindrical portion located between the second reduced-diameter portion and the second increased-diameter portion and having a cylindrical shape.

4. A bactericide diffusion method using the bactericide diffusion apparatus as recited in any one of claims 1 to 3, the method comprising:
adjusting an amount of diffusion of the bactericide from the first opening by adjusting a degree of opening of the first opening; and
housing the first container in the second container.
